# EUROPÄISCHE PATENTSCHRIFT

(11) **EP 0 858 814 B1**
(45) Veröffentlichungstag und Bekanntmachung des Hinweises auf die Patenterteilung: **10.09.2003**
(21) Anmeldenummer: 98101541.5
(22) Anmeldetag: 29.01.1998
(51) Int. Cl.: A61M 39/02

(54) **Implantierbares Doppel-Portsystem**
Implantable dual access port system
Système d'accès implantable à double entrée

(30) Priorität: 18.02.1997 DE 19706139
(43) Veröffentlichungstag der Anmeldung: 19.08.1998
(73) Patentinhaber: Tricumed Medizintechnik GmbH, 24143 Kiel (DE)
(72) Erfinder: Otto, Karl-Heinz, 24146 Kiel (DE); Kovacevic, Sasa, 23758 Oldenburg (DE)
(74) Vertreter: Tönnies, Jan G.

(56) Entgegenhaltungen:
- EP-A- 0 119 596
- EP-A- 0 230 747
- DE-A- 4 225 524
- US-A- 4 915 690

## Beschreibung

Die Erfindung betrifft einen implantierbaren Doppel-Zuspritzport zum Applizieren eines Medikaments mit den Merkmalen des Oberbegriffs des Anspruchs 1.

Aus der DE 42 25 524 A1, die zur Bildung des Oberbegriffs des Anspruchs 1 kerangezogen worden ist, ist ein implantierbares Zuspritzport mit zwei über durchstechbare Septen zugänglichen Medikamentenkammern beschrieben, bei dem das eine Septum das andere ringförmig umgibt, wobei die beiden Septen aber in einer Ebene liegen. Eine ähnlich Ausgestaltung eines Doppel-Zuspritzports zeigt die AT 391 416 B.

Aus der EP 0230 747 A2 ist ein Port mit einem seitlichen Septum bekannt, das ein flaches Anlegen einer Injektionsnadel auf der Haut des Patienten ermöglicht.

Der Erfindung liegt die Aufgabe zugrunde, ein implantierbares Doppel-Zuspritzport zu schaffen, bei dem eine Dauerinfusion über eine bei Verwendung im wesentliche auf der Haut des Patienten aufliegenden Injektionsnadel möglich ist, bei dem über eine zweite Medikamentenkammer ein weiteres Medikament infundiert werden kann oder aber auch während der laufenden Dauerinfusion Blut dem Körper des Patienten entnommen werden kann, und bei dem ein versehentliches Eindringen der Injektionsnadel in die falsche Medikamentenkammer verhindert wird.

Erfindungsgemäß wird diese Aufgabe durch die Merkmale des Anspruchs 1 gelöst, Anspruch 2 gibt eine bevorzugte Ausgestaltung der Erfindung an.

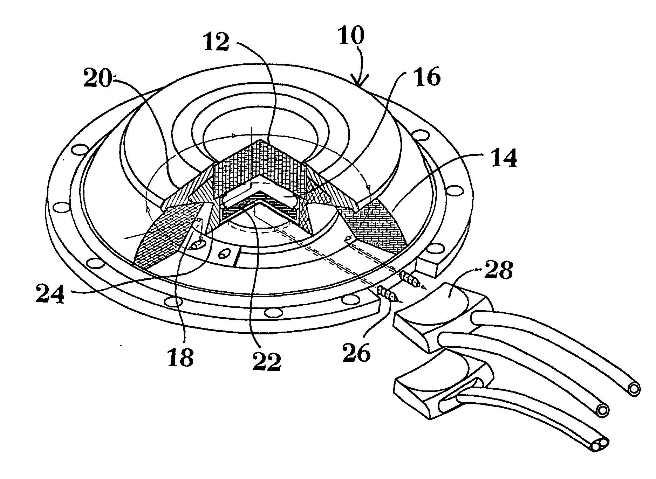

Die Erfindung wird anhand einer Zeichnung erläutert. Dabei zeigt die einzige Figur eine perspektivische, teilweise geschnitte Ansicht eines Ausführungsbeispiels eines solchen Doppel-Zuspritzports.

Der Doppel-Zuspritzport besteht aus einem linsenförmigen Gehäuse 10 mit einer nach der Implantation dicht unter der Haut des Patienten liegenden oberen Fläche, zwei von einer - nicht gezeigten - Injektionsnadel durchdringbaren Septen 12, 14, zwei den Septen 12, 14 zugeordneten Medikamentenkammern 16, 18 und mit zwei zum Anschluß eines Katheters dienendenen Auslässen. Um das Gehäuse 10 herum läuft ein mit Löchern versehener Nahtring, der zum Annähen des Doppel-Zuspritzports am Gewebe des Patienten dient. Das eine Septum 12 ist mittig in die obere Fläche des Gehäuses 10 eingebracht, das andere Septum 14 läuft an der Seite des Gehäuses 10 um diesen herum. Zwischen den beiden Septen 12, 14 ist eine Ringscheibe 20 aus einem von einer Injektionsnadel nicht durchdringbaren Material angeordnet.

Unterhalb des einen Septums 12 ist eine erste Medikamentenkammer 16 angeordnet, die auf ihrem Boden von einer Nadelstoppscheibe 22 abgeschlossen wird. Eine zweite Medikamentenkammer 18 ist als unterhalb des anderen Septums 14 umlaufender Ringraum abgebildet, der nach innen durch einen Nadelstoppring 24 abgeschlossen wird. Die Medikamentkammern 16, 18 kommunizieren mit Hohlstiften 26, die bei Einsetzen eines mit einem Buchsenstecker 28 versehenen Katheter in die - in der Zeichnung nicht erkennbaren - Buchsen des Buchsensteckers 28 eingreifen. Die beiden in der Zeichnung dargestellten Katheter können wahlweise verwendet werden.

## Patentansprüche

1. Implantierbarer Doppel-Zuspritzport zum Applizieren eines Medikaments, mit einem im wesentlichen linsenförmigen, mit einer nach der Implantation dicht unter Haut des Patienten liegenden oberen Fläche und einer umlaufenden Seitenfläche versehenen Gehäuse (10), das ein mittig angeordnetes erstes Septum (12) und ein um das erste Septum (12) herum ringförmig angeordnetes zweites Septum (14) sowie zwei den Septen (12, 14) zugeordnete Medikamentenkammern (16, 18) und zwei zum Anschluß eines Katheters dienende Auslässe aufnimmt,
**dadurch gekennzeichnet, daß** das zweite Septum (14) ringförmig in der Seitenfläche des Gehäuses (10) um dieses umlaufend ausgebildet ist und zwischen den beiden Septen (12, 14) eine Ringscheibe (20) aus einem von einer Injektionsnadel nicht durchdringbaren Material angeordnet ist.

2. Implantierbarer Dopppel-Port nach Anspruch 1, **gekennzeichnet durch** eine Ausbildung der Auslässe und des Katheters als Stecker/Buchsen-Anordnung.

## Claims

1. Implantable dual injection port for the administration of a medicament, with a substantially lenticular housing (10) with an upper surface located just below the skin of the patient following implantation and a circumferential lateral surface, said housing having a centrally positioned, first septum (12) and a second septum (14) arranged in annular manner around the first septum (12), as well as two medicament chambers (16, 18) associated with the septa (12, 14) and two outlets used for the connection of a catheter, **characterized in that** the second septum (14) is constructed in annular manner passing circumferentially around the lateral surface of the housing (10) and between the two septa (12, 14) is provided an annular disk (20) made from a material not penetratable by a hypodermic needle.

2. Implantable dual injection port according to claim 1, **characterized by** a construction of the outlets and the catheter as a plug/socket arrangement.

## Revendications

1. Système d'injection implantable, à double entrée, pour l'administration d'un médicament, avec un boîtier (10) essentiellement en forme de lentille, présentant une surface supérieure qui se trouve juste sous la peau du patient après l'implantation, et une surface latérale en circuit fermé, lequel boîtier contient un premier septum (12), disposé au centre, et un deuxième septum (14) disposé en forme d'anneau autour du premier septum (12), ainsi que deux chambres à médicament (16, 18), associées aux septums (12, 14), et deux ouvertures destinées au raccordement d'un cathéter,
**caractérisé en ce que** le deuxième septum (14) est conçu en forme d'anneau dans la surface latérale du boîtier (10) qu'il entoure et qu'une pièce annulaire (20), en matière résistante à la perforation par la canule d'injection, est disposée entre les deux septum (12, 14).

2. Système d'injection transplantable à double entrée selon la revendication 1,
**caractérisé par** une conception des ouvertures et du cathéter en tant qu'assemblage à fiches mâles / femelles.
